# EUROPEAN PATENT APPLICATION

(11) **EP 0 783 104 A1**
(43) Date of publication of application: **09.07.1997**
(21) Application number: 96120269.4
(22) Date of filing: 17.12.1996
(51) Int. Cl.: G01N 33/68, G01N 33/577, C07K 16/18, C07K 14/47

(54) **Method for assaying soluble amyloid precursor protein**

(30) Priority: 27.12.1995 JP 351296/95
(71) Applicant: ORIENTAL YEAST CO., LTD., Tokyo (JP)
(72) Inventor: Taniguchi, Yoshiyuki, Osaka-fu (JP); Fujita, Tuyosi, Ikeda-shi, Osaka-fu (JP); Matuo, Yuhsi, Suita-shi, Osaka-fu (JP)
(74) Representative: Thomsen, Dieter, Dr.

(57) **Abstract**

The present invention relates to a method for assaying soluble APP, comprising using an antibody against amyloid β protein (Aβ) or amyloid precursor protein (APP).
In accordance with the present invention, soluble APP can be accurately assayed. Additionally, an assay system composed of a monoclonal antibody recognizing the N-terminus of Aβ and a monoclonal antibody recognizing soluble APP in combination can definitely diagnose the presence or absence of Alzheimer disease.

## Description

### Detailed Description of the Invention

### Field of the Invention

The present invention relates to an assay system of amyloid precursor protein (sometimes referred to as "APP" hereinbelow), in particular soluble amyloid precursor protein (sometimes referred to as "sAPP" hereinafter).

The substance sAPP is involved in the generation of amyloid β protein (sometimes referred to as "Aβ" hereinafter) as a component of senior plaque frequently observed in patients with Alzheimer disease. The present invention is highly promising as a useful diagnostic system for Alzheimer disease.

### Prior Art

Alzheimer disease, one of the diseases involving dementia due to the denaturation of nervous cells, is observed in the early stage of aging and thereafter, and therefore, the disease has been drawing social concerns in recent years with the increase in the aged population. A great number of senior plaque is observed in the brain of Alzheimer patients, and the senior plaque is primarily composed of Aβ.

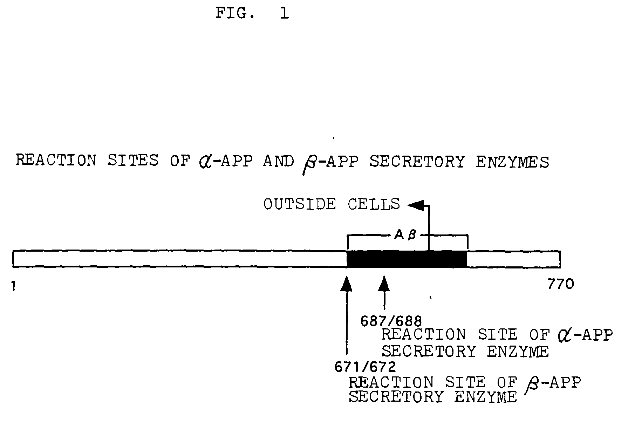

A part of the amyloid precursor protein (APP) is cleaved at the proximity of the cell transmembrane region with an APP secretory enzyme to generate Aβ, which is a protein of 39 to 43 amino acids. Fig. 1 schematically depicts the site of the membrane-binding type APP with which the APP secretory enzyme reacts.

α- and β-APP secretory enzymes cleave APP at the sites shown in the figure to discharge α-sAPP and β-sAPP, respectively, outside the cells. It is suggested that after discharge of sAPP, the C-terminus of APP, left on the cell membrane, is incorporated into the inside of the cells and is then decomposed with lysozyme. For processing with the β-APP secretory enzyme, the entire length of Aβ remains on the cell membrane so that the entire length of Aβ is secreted through the decomposition with lysozyme. For processing with the α -APP secretory enzyme, alternatively, the amino acid sequence of the first to the 16-th amino acids from the N-terminus of Aβ is released as a part of sAPP. Therefore, the complete form of Aβ is not secreted. In other words, because of the processing of APP with the α-APP secretory enzyme, APP is metabolized with no generation of Aβ which is the component of senior plaque.

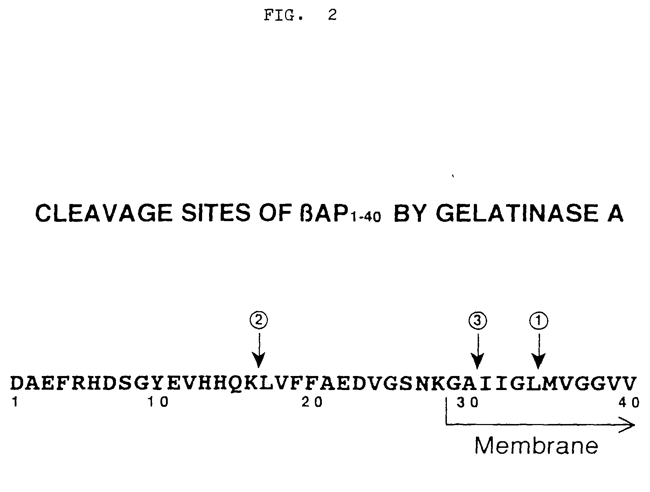

Fig. 2 depicts the amino acid sequence of Aβ(1-40), composed of 40 amino acids. In the figure, the arrow shows the cleavage site between the 16th amino acid (Lys) and the 17th amino acid (Leu) from the N-terminus.

Herein, APP is present in various isomers, primarily including APP 770 described above, APP 751 similarly carrying the Knitz-type protease inhibitor region (KPI region), and APP 695 without the KPI region, all of them being known. In accordance with the present invention, the term "APP" includes all these isomers, but APP 770 is used as one representative example hereinbelow to describe the present invention.

A method for assaying sAPP comprising the combination of Western blotting and immunostaining by the use of antibody and measuring the spot detected on the blotting membrane by means of a scanner, has been examined. From the respect of antibody reactivity, the substance herein assayed is possibly the total of α-sAPP and β-sAPP, but no apparent difference has been found between the sAPP level in clinical samples from normal subjects and that level in samples from Alzheimer patients.

Lannfeld et al. recently carried out the assay of α-sAPP by using the combination of Western blotting and immunostaining by using an antibody reactive with the amino acid sequence of the first to the 15-th amino acids of Aβ. Consequently, they indicated that α-sAPP might possibly serve as a highly specific diagnostic marker of Alzheimer disease, because the level of α-sAPP is significantly lower in the cerebro-spinal fluid of a carrier group with the variation in the amino acid sequence of APP 670/671 known as one of the genetic variants as the etiology of familial Alzheimer disease than the level in the cerebro-spinal fluid of a normal group (Lannfeld L. et al., Nature medicine 1, 829-832 (1995) ). However, the assay procedure in the report is very complex, comprising electrophoretic separation of a sample as described above, transfer of the protein on a membrane by Western blotting, immunostaining with an antibody and the detection and assay of α-sAPP by means of a scanner.

### Problems to be Solved by the Invention

It is believed that sAPP, in particular α-sAPP from the cleavage with the α-APP secretory enzyme, may potentially be a marker effective for diagnosis of Alzheimer disease, but because the sAPP assay procedure by means of the combination of Western blotting and immunostaining with an antibody as described above is very complex, the sAPP assay is not suitable for practical use. Thus, the inventors have established an sAPP assay system capable of assaying sAPP in a simple manner by using an antibody against sAPP. The assay system provides a practical method for diagnosis of Alzheimer disease.

### Brief Description of Drawings

Fig. 1 depicts the reaction sites of Aβ with α- and β-APP secretory enzymes;
Fig. 2 depicts the amino acid sequence of Aβ (1-40); and
Fig. 3 shows the results of sAPP assay.

### Means for Solving the Problems

So as to overcome the problems described above, the present inventors have made investigations to prepare a plurality of antibodies against sAPP and establish the immunoassay of sAPP by means of their combinations.

The preparation of antibodies to be used for such assay is carried out according to already known methods. More specifically, immunizing an animal with an immunogen sAPP or a peptide having a part of the amino acid sequence of sAPP, a polyclonal antibody having the reactivity with sAPP is generated in the serum. More preferably, resecting the spleen of the immunized animal, hybridizing an antibody-generating cell contained in the spleen with a myeloma cell to prepare a hybridoma followed by cloning, a homogeneous monoclonal antibody can be recovered at a large scale.

The immunogen to be used for immunizing animals is purified from the culture supernatant of an established cell line from a human nervous cell; otherwise, a part of human APP is prepared by peptide synthesis on the basis of the well known amino acid sequence of human APP, which is then used as such immunogen.

Animals such as goat, sheep, horse, rabbit, rat, and mouse are used for immunization. So as to prepare a monoclonal antibody, generally, mouse or rat is used from the respect of the compatibility with myeloma cells for fusion.

For immunization, an immunogen may be mixed with a variety of adjuvants so as to promote immune response; particularly when a synthetic peptide is to be used as such immunogen, the peptide preliminarily should be cross-linked with carrier proteins such as BSA (bovine serum albumin) and KLH (keyhole limpet hemocyanine) for such use.

The preparation of a hybridoma generating a monoclonal antibody is carried out according to the method described in Koehler, G. and Milstein, C., Nature 256, 495 (1975). More specifically, a splenocyte resected from an immunized animal is mixed and fused with a myeloma cell in the presence of polyethylene glycol. The hybridoma generated is selectively grown in a HAT medium (containing 1 × 10⁻⁴ M hypoxanthine, 4 × 10⁻⁷ M aminopterin, and 1.6 × 10⁻³ M thymidine), and the cell involving the secretion of an antibody into the culture supernatant under observation is cloned by the limited dilution method.

The thus prepared hybridoma can generate a monoclonal antibody from the culture supernatant or the ascites fluid recovered from the transplant of the hybridoma into the animal from which the myeloma cell is derived.

The anti-serum of the immunized animal, the culture supernatant of the hybridoma, and the antibody from the ascites fluid may be purified by known purification methods such as ammonium sulfate salting out, ion exchange chromatography, and affinity chromatography.

The immunoassay of sAPP in accordance with the present invention comprises immobilizing one antibody onto an insoluble carrier, capturing a subjective assaying substance in a sample onto the antibody, thereafter reacting the other labeling substance-bound antibody with the subjective substance and detecting the activity of the labeling substance bound onto the insoluble carrier thereby assaying the subjective substance. The insoluble carrier to be used for immobilizing the antibody includes those of forms such as tube, beads, ball, and well, and as the material thereof, use may be made of plastics, glass, silicon, and latex.

Alternatively, the labeling substance is bound to the antibody to detect the antigen captured on the insoluble carrier. The labeling substance includes enzyme, radioactive substance, fluorescent substance, luminescent substance and the like, and the assay of the activity of the labeling substance can yield the measurement of the level of the antigen captured.

If desired, still further, biotin may be bound to the antibody to prepare a biotin-labeled antibody, with which avidin labeled with a labeling substance reacts to assay the antigen level, in addition to the direct binding of the labeling substance to the antibody.

So as to carry out the assay method of the present invention, for example, an anti-sAPP antibody is immobilized on a carrier, and onto the immobilized antibody is captured the sAPP antigen in a sample, and a labeled anti-Aβ antibody reacts with the resulting antigen-antibody complex. If the amino acid sequence of the sAPP antigen contains the amino acid sequence corresponding to Aβ (for example, α-sAPP), the labeled anti-A β antibody is bound to the sAPP antigen. Then, by activating and assaying the labeling substance through color development or fluorescent emission, the sAPP antigen is assayed quantitatively and qualitatively.

The present invention is to provide not only an assay method of sAPP, but also an sAPP assay kit including a variety of antibodies, buffer solutions, labeling and activating reagents, and other necessary substances to be used for the assay, and a kit for detecting Alzheimer disease, and the like. A kit incorporating an anti-sAPP monoclonal antibody and an anti-Aβ monoclonal antibody is useful for establishing the diagnosis of Alzheimer disease, and the application thereof is highly promising for health check programs for aged people.

The present invention will now be described in detail in examples hereinbelow, but these examples are not intended to limit the present invention only to sAPP derived from APP 770, excluding other isomers.

### Example 1

### Preparation of monoclonal antibodies against Aβ and sAPP

### 1. Materials

### Immunogen

Regarding Aβ, a peptide having the amino acid sequence of the first to the 40-th amino acids of Aβ was prepared by peptide synthesis, which was used as an immunogen. Regarding sAPP, culturing a human urinary bladder cancer cell EJ-1 in a serum-free RPMI 1640 medium, sAPP was purified from the culture supernatant by activated Red Sepharose column chromatography and Sephadex QA 824 column chromatography.

### 2. Immunization

The immunization procedure for preparing Aβ antibody will be described below. To a solution (0.9 ml) containing Aβ (1 mg) was added an equal amount of Freund complete adjuvant, for sufficient mixing to prepare an emulsion, which was then injected intraperitoneally at 1.8 ml/mouse into a Balb/c mouse for immunization. Then, an emulsion of a solution containing the solution (0.6 ml) containing Aβ (1 mg) and incomplete adjuvant was injected subcutaneously as an immunogen at 1.2 ml/mouse every three weeks. After such immunization procedure was repeated several times, a PBS solution containing Aβ (100 µg) was boosted intraperitoneally into the mouse at 0.2 ml/mouse as the final immunization.

The immunization procedure for preparing sAPP antibody will be described below. To a solution (0.75 ml) containing sAPP (150 µg) was added an equal amount of Freund complete adjuvant, for sufficient mixing to prepare an emulsion, which was then injected intraperitoneally at 1.5 ml/mouse into a Balb/c mouse for immunization. Then, an emulsion of a solution containing the solution (0.4 ml) containing sAPP (75 µg) and incomplete adjuvant was injected as an immunogen subcutaneously at 0.8 ml/mouse every three weeks. After such immunization procedure was repeated several times, a PBS solution containing sAPP (62.5 µg) was boosted intraperitoneally into the mouse at 0.3 ml/mouse as the final immunization.

### 3. Cell fusion

### Fusion

Three days after the final immunization, spleen was aseptically resected from the mouse, which was finely chopped into small pieces for cell extrusion. The extruded cells after passing through nylon mesh were then washed several times in an RPMI 1640 medium for re-suspension to prepare a suspension of the spleen cells. P3-X63-Ag8-653 was used as a murine myeloma cell for fusion. The myeloma cells to be used for such purpose include for example P3-X63-Ag8-U1 and P3-Ns-1/Ag4-1, which are all known and commercially available.

Cell fusion was carried out according to the method of Koehler. G. et al. More specifically, spleen cells and the suspension of murine myeloma cells, preliminarily washed three times in a serum-free RPMI 1640 medium, were mixed together at a cellular ratio of 5:1. The cells were precipitated by centrifuging to remove the supernatant medium, and the remaining cells were mildly loosened, to which was then gradually mixed 50 % polyethylene glycol 6000 (1 ml; manufactured by Boehringer, Co. Ltd.) for fusion. Furthermore, adding gradually an RPMI 1640 medium (10 ml) containing 10 % serum to dilute the resulting mixture followed by centrifuging, the resulting supernatant was discarded. The cells were suspended in a HAT medium (50 ml) containing 1 × 10⁻⁴ M hypoxanthine, 4 × 10⁻⁷ M aminopterin, and 1.6 × 10⁻³ M thymidine, and the resulting suspension was divided at 0.5 ml/well into a 48-well culturing plate.

### Antibody screening of the culture supernatant

After the initiation of the culturing in the HAT medium, the medium was exchanged to fresh such medium or fresh medium was added at an appropriate time. About 10 days later, screening of antibodies in the culture supernatant was conducted. After adding and immobilizing an antigen solution (5-10 µg/ml) into a microtiter plate for ELISA, 1 % bovine serum albumin was used for blocking. Supernatants sampled from the culture plate were added at 100 µl into the microtiter plate, which was then left to stand at room temperature for 2 hours. After washing, 100 µl of horse radish peroxidase (POD)-labeled rabbit anti-mouse IgG antibody (10 µg/ml) was added into each well, which was then left to stand at room temperature for 2 hours. After washing the plate, 100 µl each of a substrate solution(100 mM phosphate buffer solution, pH 6.0; 1 mg/ml o-phenylene diamine, 1 µl/ml H₂O₂) was added into the well for reaction for an appropriate period of time, followed by addition of 100 µl of 4N HCl to terminate the reaction. The absorbance at 492 nm of each well of the plate was measured to identify antibodies contained in each sample on the basis of the measurement.

### Cloning

Cloning of the thus recovered hybridoma by the method described above was carried out by limited dilution. For cloning, a suspension of the spleen cells from a Balb/c mouse to a final concentration of about 1 × 10⁶ cells /ml was used as a feeder cell, and the hybridoma was diluted with the feeder cell suspension to 0.5 cell/ml. The hybridoma diluting solution was divided at 0.1 ml into a 96-well culture plate, and about one week later, the formation of colonies was confirmed under microscopic observation. Then, a well of monoclones was confirmed of its antibody generation by the method described above. Single clone of each of antibody-generating hybridomas of Aβ and sAPP, was confirmed.

The thus recovered hybridomas were both new fused cells and named individually as mouse-mouse hybridoma β AP/OM16 and mouse-mouse hybridoma APP/OM84. These hybridomas were then deposited internationally as FERM BP-4673 and FERM BP-4672 in the Bioengineering and Industrial Technology Research Institute, the Agency of Industrial Science and Technology, Japan.

### Example 2

### Characterization of antibodies

The cells of the new hybridomas prepared as described above were intraperitoneally administered (1 to 3 × 10⁶ cells/mouse) to mice preliminarily given mineral oil (0.5 ml) intraperitoneally. Six to 20 days later, ascites fluid containing antibodies was collected to purify monoclonal antibodies from the ascites fluid by using a protein-A column according to a routine method.

The characterization of the generated monoclonal antibodies brings about the results as follows;
- anti-Aβ antibody;: subclass IgG2b/χ
- specificity;: reactivity with both of Aβ and sAPP
(recognizing the amino acid sequence of the first to the 16-th amino acids from the N-terminus of Aβ, the amino acid sequence being common to both of Aβ and sAPP.)
- anti-sAPP antibody;: subclass IgG2a/χ
- specificity;: specific reactivity with sAPP
(recognizing the amino acid sequence present in sAPP, excluding the amino acid sequence of the first to the 16-th amino acids from the N-terminus of Aβ; thus, recognizing β-sAPP and β-sAPP-containing substance (α-sAPP)).

### Example 3

### Antibody labeling with biotin

So as to establish the assay of sAPP using the generated antibodies, the antibodies were labeled with biotin. Firstly, the concentration of the antibodies was adjusted to about 5 mg IgG/ml, which was then dialyzed against 100 mM carbonate buffer solution, pH 9.0. A biotin-labeling reagent (NHS-LC-Biotin II, manufactured by PIERCE Company) was dissolved in dimethyl formamide to a final concentration of 30 mg/ml, which was then added at 10 µl into 1 ml of the antibody solution for thorough mixing. The resulting solution was left to stand in ice for 2 hours for reaction. The solution was then dialyzed against PBS to remove the unreactive biotin-labeling reagent.

### Example 4

### Assaying of soluble APP

Anti-sAPP antibody was diluted to 10 µg/ml by using PBS, which was then divided at 50 µl each into a 96-well microtiter plate, and the plate was left to stand overnight at 4 °C. After discarding the solution in the wells, each well was filled with PBS containing 0.1 % BSA (0.1 % BSA-PBS) and was then left to stand at room temperature for 3 hours. Each well was washed in PBS containing 0.1 % Tween-20 (0.1 % Tween-PBS) five times, to which was then added 50 µl of sAPP antigen diluted with 0.1 % BSA-PBS to 10, 5, 1, 0.5, 0.1, 0.05, and 0.01 µg/ml. Each well was left to stand at room temperature for 2 hours, prior to washing in 0.1 % Tween-PBS five times. 50 µl of each of biotin-labeled anti-Aβ antibodies diluted to 10 µg/ml by using 0.1 % Tween-PBS was added to each well, which was then left to stand at room temperature for 2 hours.

After washing each well five times in 0.1 % Tween-PBS, 50 µl of Vectastain ABC kit Elite PK-6100 (manufactured by Vector Company) diluted to 150- fold by using 0.1 % Tween-PBS was added to each well, which was then left to stand at room temperature for one hour. After washing each well five times in 0.1 % Tween-PBS, 100 µl of a substrate solution (100 mM phosphate buffer solution, pH 6.0, 1 mg/ml o-phenylene diamine, 1 µl/ml H₂O₂) was added to each well for reaction at room temperature for an appropriate period of time, followed by addition of 100 µl of 4N HCl to each well to terminate the reaction. The absorbance of each well at 492 nm was measured with a plate reader.

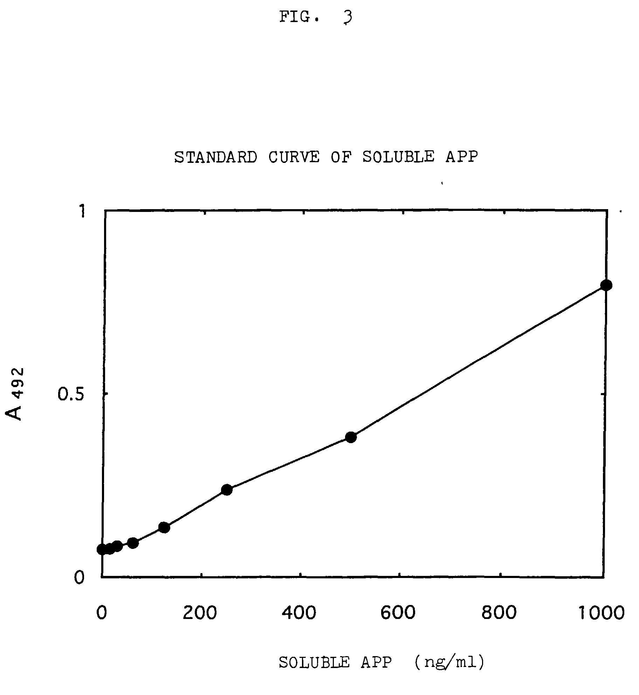

The results of the measurement are shown in Fig. 3. The results indicate that sAPP antigen level can be measured accurately.

### Effects of the Invention

In accordance with the present invention, sAPP can be measured accurately at a higher sensitivity in a simple manner. An immunoassay system composed of the anti-sAPP monoclonal antibody and the anti-Aβ monoclonal antibody in combination can screen accurately Alzheimer patients, so the immunoassay system can be effectively used for diagnosis of Alzheimer disease.

## Claims

1. A method for assaying soluble APP, comprising using an antibody against amyloid β-protein or soluble amyloid precursor protein (APP).

2. A method for assaying soluble APP according to claim 1, using an antibody having the antigen recognition site which is the amino acid sequence in common to amyloid β-protein and soluble APP or which is a specific amino acid sequence to soluble APP.

3. A method for assaying soluble APP according to any one of claims 1 and 2, wherein the assaying subject is soluble APP having a part of the amino acid sequence of amyloid β-protein at the amino terminus thereof.

4. A method for assaying soluble APP according to any one of claims 1 to 3, wherein the assaying subject is APP solubilized through the cleavage of the amyloid β-protein between the 16-th lysine and the 17-th leucine from the amino terminus thereof.

5. A method for assaying soluble APP according to any one of claims 1 to 4, comprising immobilizing one antibody onto an insoluble carrier, capturing a subjective assaying substance in a sample onto the antibody, thereafter reacting the other labeling substance-bound antibody with the subjective substance and detecting the activity of the labeling substance bound onto the insoluble carrier thereby assaying the subjective substance.

6. A method for assaying soluble APP according to any one of claims 1 to 5, wherein soluble APP is measured for diagnosing Alzheimer disease.

7. A method for assaying soluble APP according to any one of claims 1 to 6, wherein the antibody to be used is a monoclonal antibody.

8. A monoclonal antibody specifically recognizing the amino acid sequence of amyloid β-protein from the first to the 16-th amino acids from the amino terminus thereof.

9. A monoclonal antibody recognizing a part of the amino acid sequence of amyloid β protein present on soluble APP, excluding the amino acid sequence thereof from the first to the 16-th amino acids from the amino terminus thereof.

10. A method for assaying soluble APP according to claim 7, wherein the monoclonal antibody is a monoclonal antibody according to claims 8 or 9.

11. Hybridoma cells, mouse-mouse hybridoma β AP/OM16 and mouse-mouse hybridoma APP/OM84, generating a monoclonal antibody according to claims 8 or 9, and the generated antibodies.

12. A method for assaying soluble APP, comprising immobilizing one antibody of the monoclonal antibodies generated by hybridoma cells according to claim 11 onto an insoluble carrier, capturing a subjective assaying substance in a sample onto the antibody, thereafter reacting the other labeling substance-bound antibody with the subjective substance and detecting the activity of the labeling substance bound onto the insoluble carrier thereby assaying the subjective substance.

13. A kit for detecting Alzheimer disease, containing a monoclonal antibody according to claims 8 or 9.
